# EUROPEAN PATENT APPLICATION

(11) **EP 2 896 398 A1**
(43) Date of publication of application: **22.07.2015**
(21) Application number: 13837514.2
(22) Date of filing: 10.09.2013
(51) Int. Cl.: A61K 33/26, A61K 9/10, A61K 9/16, A61K 47/32, C01G 49/00, C01G 49/06, A61N 1/40, A61N 2/00, A61N 5/02

(54) **SURFACE-MODIFIED IRON OXIDE PARTICLES FOR CANCER CAUTERIZATION**

(30) Priority: 12.09.2012 JP 2012200836
(71) Applicant: University of Tsukuba, Tsukuba-shi, Ibaraki 305-8577 (JP)
(72) Inventor: ODA, Tatsuya, Tsukuba-shi Ibaraki 305-8577 (JP); NAGASAKI, Yukio, Tsukuba-shi Ibaraki 305-8577 (JP); ASAI, Kei, Tsukuba-shi Ibaraki 305-8577 (JP); KITA, Eiji, Tsukuba-shi Ibaraki 305-8577 (JP); YANAGIHARA, Hideto, Tsukuba-shi Ibaraki 305-8577 (JP); OHKOHCHI, Nobuhiro, Tsukuba-shi Ibaraki 305-8577 (JP); KISHIMOTO, Mikio, Tsukuba-shi Ibaraki 305-8577 (JP)
(74) Representative: Louis Pöhlau Lohrentz
(86) International application number: PCT/JP2013/074350
(87) International publication number: WO 2014/042142

(57) **Abstract**

An object of the present invention is to provide surface-modified ferromagnetic iron oxide particles suitable for electromagnetic-induction cancer ablation, which have excellent heat generation properties and also have excellent dispersion stability in a medium for injection. The surface-modified iron oxide particles for cancer ablation of the present invention as a means for achieving the object are characterized in that a block copolymer of polyethylene glycol and polystyrene having a phosphorous acid group in the side chain is bound to the surface of ferromagnetic iron oxide particles having a plate-like shape with a length of 20 to 200 nm and a length-to-thickness ratio of 1.5 to 30 and having magnetic properties such that the coercivity is 30 to 300 Oe, the saturation magnetization is 20 to 80 emu/g, and the squareness ratio of the magnetic hysteresis loop is 0.20 to 0.50.

## Description

### Technical Field

The prevent invention relates to surface-modified iron oxide particles for cancer ablation. More specifically, the present invention relates to surface-modified ferromagnetic iron oxide particles suitable for electromagnetic-induction cancer ablation, which have excellent heat generation properties and also have excellent dispersion stability in a medium for injection.

### Background Art

"Heat" is the most reliable tool to kill cancer cells. It is a long known fact that when an amount of heat that causes irreversible thermal denaturation to a tumor-forming protein (e.g., 50°C or more x 10 minutes) is applied to the tumor, the cancer nodules disappear. In actual clinical practice, it has been common to directly puncture the lesion with a needle electrode and thermally ablate the cancer with radio waves, etc., for example. However, it often happens that the lesion cannot be entirely heated to a high temperature, and some parts are left unburned, rendering the treatment unsuccessful. Further, in many of refractory cancers and advanced cancers, there are countless cancer lesions of various sizes from several tens of micrometers to several centimeters, and it is realistically impossible to directly puncture each of the cancer lesions with a needle electrode and heat them. Thus, not all cancers can be subjected to puncture ablation.

The idea of a treatment method, in which magnetic particles are accumulated in cancer cells, and then an alternating current magnetic field (electromagnetic wave) is applied from outside the body to perform induction heating, thereby killing the cancer cells, has been advocated over 20 years. In order for this treatment method to be clinically realized, for example, in the case of the ablation of a tumor 1 cm in diameter, it is necessary to apply 1 W or more of heat per g of tumor. The amount of heat is determined by the product of the calorific value of magnetic particles and the amount of particles accumulated in the tumor. At present, it is believed that the amount of magnetic particles that can be accumulated in cancer cells is technically limited to 1 to 2 mg/g of tumor. Therefore, the development of magnetic particles having a calorific value of not less than 500 W/g of magnetic particles, preferably not less than 1,000 W/g of magnetic particles, has been a challenge in treating refractory cancers and advanced cancers that cannot be dealt with by puncture ablation.

Magnetic particles that have been mainly used in the study of electromagnetic-induction cancer ablation are dextran magnetite. Dextran magnetite is a sensitizer to be injected into humans for sensitizing effects in nuclear magnetic resonance imaging (MRI) and is commercially available under the trade name Resovist. Dextran magnetite has excellent dispersion stability in a medium for injection and thus can be administered safely to humans. However, these magnetic particles are nanoparticles having a particle size of 10 nm or less, and their magnetic properties are classified as superparamagnetic. Therefore, efficient heat generation using magnetic hysteresis loss cannot be expected, and the overall calorific value is only 32 W/g of magnetic particles when exposed to a magnetic field of 120 kHz, 480 Oe. Accordingly, the heat generation efficiency of dextran magnetite is essentially low, and thus, currently, it has not been put into practical use as magnetic particles in electromagnetic-induction cancer ablation.

In view of the above points, in recent years, a method in which ferromagnetic iron oxide particles are introduced into the body and accumulated in cancer cells, and then an alternating current magnetic field is externally applied to generate heat by magnetic hysteresis loss, thereby ablating the cancer, has been studied (e.g., Patent Document 1). This method using ferromagnetic iron oxide particles is advantageous in that heat can be efficiently generated at a relatively low frequency, and it is believed that a calorific value of not less than 500 W/g of magnetic particles can be achieved. Therefore, the particles are expected to be put into practical use as magnetic particles in electromagnetic-induction cancer ablation. However, the heat generation efficiency currently achieved is not necessarily satisfactory, and intensive studies have been conducted to improve the heat generation efficiency. Further, a fundamental problem in using the ferromagnetic iron oxide particles for electromagnetic-induction cancer ablation is that because the particles tend to aggregate and sediment when dispersed in a medium for injection, it is difficult to obtain a dispersion having excellent dispersion stability. This is because, unlike superparamagnetic particles, ferromagnetic particles themselves have properties as magnets, and thus strong magnetic cohesive force is exerted between particles in a medium.

As a method for achieving the improvement of the heat generation efficiency of ferromagnetic iron oxide particles, which is the first problem in using ferromagnetic iron oxide particles as magnetic particles for electromagnetic-induction cancer ablation, for example, a possible method is to increase the coercivity or squareness ratio (the ratio of remanent magnetization to saturation magnetization) of the magnetic hysteresis loop to increase the magnetic hysteresis loss. However, in a method that increases the coercivity, although the area of the hysteresis loop increases, it is necessary to apply a high magnetic field in accordance with the increased coercivity, and thus the equipment will be large-scale and expensive. Therefore, this method is not practical. Meanwhile, a method that increases the squareness ratio does not require large-scale and expensive equipment and thus can be regarded as a practical method. However, ferromagnetic iron oxide particles heretofore proposed have a spherical or elliptical shape. Therefore, in order to increase the squareness ratio of the magnetic hysteresis loop, it is necessary to add foreign elements, such as cobalt. According to such a method in which a foreign element is added, usually, together with an increase in the squareness ratio, the coercivity also increases. Therefore, it is required to increase only the squareness ratio without increasing the coercivity, but there has been no material or method that can realize it, and thus the improvement of heat generation efficiency has been limited. Thus, the present inventors conducted research for the purpose of increasing the squareness ratio of a magnetic hysteresis loop to increase the magnetic hysteresis loss, thereby providing ferromagnetic iron oxide particles for cancer ablation with excellent heat generation properties. Then, they have reported in Nonpatent Document 1 that ferromagnetic iron oxide particles having a plate-like shape produced via goethite (α-FeOOH) using a trivalent iron compound as a starting material are excellent and satisfy the above requirements.

### Prior Art Documents

### Patent Documents

Patent Document 1: JP-A-2010-89991

### Nonpatent Documents

Nonpatent Document 1: M. Kishimoto et al., Journal of Magnetism and Magnetic Materials, 324 (2012), 1285-1289

### Summary of the Invention

### Problems that the Invention is to Solve

The ferromagnetic iron oxide particles having a plate-like shape reported by the present inventors in Nonpatent Document 1 have excellent heat generation properties suitable for electromagnetic-induction cancer ablation. Thus, the present inventors believe that the first problem in using ferromagnetic iron oxide particles for electromagnetic-induction cancer ablation has been solved. However, with respect to dispersion stability in a medium for injection, which is the second problem in using ferromagnetic iron oxide particles for electromagnetic-induction cancer ablation, these particles cannot overcome the magnetic cohesive force exerted between particles. Thus, the solution thereof is necessary. As a method for solving this problem, a method in which particles are surface-modified with a surface modifier made of various organic substances or inorganic substances to create a steric hindrance on the particle surface, thereby blocking the magnetic cohesive force exerted between particles in a medium, is known. Thus, the present inventors attempted to surface-modify particles using a silane coupling agent as a surface modifier. However, even when particles were surface-modified with a silane coupling agent, due to the weak binding force or the small binding amount, a steric hindrance enough to sufficiently block the magnetic cohesive force exerted between particles was not obtained, and, as a result, satisfactory dispersion stability was not obtained. Accordingly, an improved method for surface-modifying particles has been demanded. In addition, in the case where the medium for injection is physiological saline containing positively charged sodium ions and negatively charged chlorine ions, for example, these ions adhere to the particle surface and promote the aggregation of particles. Accordingly, in order for the particles to have excellent dispersion stability in an electrically charged medium, a surface modification method capable of blocking the magnetic cohesive force exerted between particles and also the cohesive force due to electrical charges is necessary.

Thus, an object of the present invention is to provide surface-modified ferromagnetic iron oxide particles suitable for electromagnetic-induction cancer ablation, which have excellent heat generation properties and also have excellent dispersion stability in a medium for injection.

### Means for Solving the Problems

In view of the above points, the present inventors have conducted extensive research. As a result, they have found that when a block copolymer of polyethylene glycol and polystyrene having a phosphorous acid group in the side chain is bound as a surface modifier to the surface of the ferromagnetic iron oxide particles having a plate-like shape reported by the present inventors in Nonpatent Document 1, while maintaining the excellent heat generation properties of the ferromagnetic iron oxide particles having a plate-like shape, the particles can be provided with excellent dispersion stability in a medium for injection.

Surface-modified iron oxide particles for cancer ablation according to the present invention accomplished based on the above findings are, as defined in claim 1, characterized in that a block copolymer of polyethylene glycol and polystyrene having a phosphorous acid group in the side chain is bound to the surface of ferromagnetic iron oxide particles having a plate-like shape with a length of 20 to 200 nm and a length-to-thickness ratio of 1.5 to 30 and having magnetic properties such that the coercivity is 30 to 300 Oe, the saturation magnetization is 20 to 80 emu/g, and the squareness ratio of the magnetic hysteresis loop is 0.20 to 0.50.

The surface-modified iron oxide particles for cancer ablation as defined in claim 2 are characterized in that in the surface-modified iron oxide particles for cancer ablation according to claim 1, the iron oxide is magnetite, gamma iron oxide, or an intermediate iron oxide between magnetite and gamma iron oxide.

The surface-modified iron oxide particles for cancer ablation as defined in claim 3 are characterized in that in the surface-modified iron oxide particles for cancer ablation according to claim 1, the ferromagnetic iron oxide particles have a BET specific surface area of 30 m²/g or more.

The surface-modified iron oxide particles for cancer ablation as defined in claim 4 are characterized in that in the surface-modified iron oxide particles for cancer ablation according to claim 1, the block copolymer of polyethylene glycol and polystyrene having a phosphorous acid group in the side chain is a polyethylene glycol-b-poly(4-vinylbenzylphosphonate) represented by the following general formula (I).

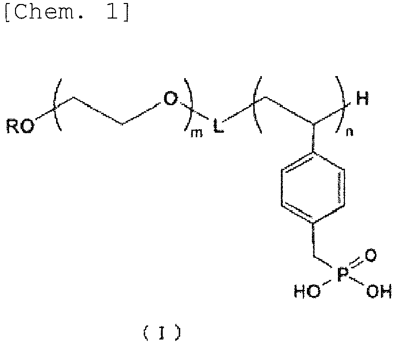

In the formula, R is an optionally substituted alkyl group, L is a linking group selected from the group consisting of -(CH₂)ₗS-, -CO(CH₂)ₗS-, -COO(CH₂)ₗS-, and -COC (CH₃)₂-, l is an integer of 1 to 5, m is an integer of 5 to 500, and n is an integer of 2 to 30.

The surface-modified iron oxide particles for cancer ablation as defined in claim 5 are characterized in that in the surface-modified iron oxide particles for cancer ablation according to claim 1, the block copolymer has a number average molecular weight of 1,000 to 30,000.

A method for producing surface-modified iron oxide particles for cancer ablation according to the present invention is, as defined in claim 6, characterized in that a block copolymer of polyethylene glycol and polystyrene having a phosphorous acid group in the side chain is dissolved in a dispersion of ferromagnetic iron oxide particles in water and/or a hydrophilic solvent as a dispersion medium, thereby binding the block copolymer to the surface of the ferromagnetic iron oxide particles,
the ferromagnetic iron oxide particles having a plate-like shape with a length of 20 to 200 nm and a length-to-thickness ratio of 1.5 to 30 and having magnetic properties such that the coercivity is 30 to 300 Oe, the saturation magnetization is 20 to 80 emu/g, and the squareness ratio of the magnetic hysteresis loop is 0.20 to 0.50.

The production method as defined in claim 7 is characterized in that in the production method according to claim 6, the binging of the block copolymer to the surface of the ferromagnetic iron oxide particles is performed at a temperature of 30 to 60°C.

The production method as defined in claim 8 is characterized in that in the production method according to claim 6, the ferromagnetic iron oxide particles are produced by subjecting a precipitate obtained by mixing an alkali and an amine compound with an aqueous solution of a trivalent iron compound to a hydrothermal reaction to produce goethite particles, followed by converting goethite into an iron oxide.

The production method as defined in claim 9 is characterized in that in the production method according to claim 8, the amine compound is an alcohol amine.

A surface-modified iron oxide particle dispersion composition for cancer ablation according to the present invention is, as defined in claim 10, characterized in that surface-modified iron oxide particles for cancer ablation made of ferromagnetic iron oxide particles having bound to the surface thereof a block copolymer of polyethylene glycol and polystyrene having a phosphorous acid group in the side chain are dispersed in a medium for injection,
the ferromagnetic iron oxide particles having a plate-like shape with a length of 20 to 200 nm and a length-to-thickness ratio of 1.5 to 30 and having magnetic properties such that the coercivity is 30 to 300 Oe, the saturation magnetization is 20 to 80 emu/g, and the squareness ratio of the magnetic hysteresis loop is 0.20 to 0.50.

### Advantage of the Invention

The present invention enables the provision of surface-modified ferromagnetic iron oxide particles suitable for electromagnetic-induction cancer ablation, which have excellent heat generation properties and also have excellent dispersion stability in a medium for injection.

### Brief Description of the Drawings

Fig. 1 is a TEM photograph of goethite particles having a plate-like shape obtained in <A>, Step A-1, of Example 1.
Similarly, Fig. 2 is a TEM photograph of gamma iron oxide particles having a plate-like shape obtained in <A>, Step A-4.
Similarly, Fig. 3 is a graph showing the heat generation properties of the gamma iron oxide particles having a plate-like shape obtained in <A>, Step A-4.
Fig. 4 is a TEM photograph of goethite particles having a plate-like shape obtained in Example 2.
Similarly, Fig. 5 is a TEM photograph of gamma iron oxide particles having a plate-like shape.

### Mode for Carrying Out the Invention

The surface-modified iron oxide particles for cancer ablation of the present invention are characterized in that a block copolymer of polyethylene glycol and polystyrene having a phosphorous acid group in the side chain is bound to the surface of ferromagnetic iron oxide particles having a plate-like shape with a length of 20 to 200 nm and a length-to-thickness ratio of 1.5 to 30 and having magnetic properties such that the coercivity is 30 to 300 Oe, the saturation magnetization is 20 to 80 emu/g, and the squareness ratio of the magnetic hysteresis loop is 0.20 to 0.50.

First, the following will describe the ferromagnetic iron oxide particles having a plate-like shape with a length of 20 to 200 nm and a length-to-thickness ratio of 1.5 to 30 and having magnetic properties such that the coercivity is 30 to 300 Oe, the saturation magnetization is 20 to 80 emu/g, and the squareness ratio of the magnetic hysteresis loop is 0.20 to 0.50. The ferromagnetic iron oxide particles having a plate-like shape are known from Nonpatent Document 1.

The kind of iron oxide is not particularly limited as long as it is a ferromagnetic iron oxide that can be used for cancer ablation. Examples thereof include magnetite (Fe₃O₄), gamma iron oxide (γ-Fe₂O₃), and an intermediate iron oxide between magnetite and gamma iron oxide.

The ferromagnetic iron oxide particles can be produced by subjecting a precipitate obtained by mixing an alkali and an amine compound with an aqueous solution of a trivalent iron compound to a hydrothermal reaction to produce goethite particles, followed by converting goethite into an iron oxide. This production method is based on the findings of the present inventors that when a precipitate (containing iron hydroxide) obtained by mixing an alkali and an amine compound with an aqueous solution of a trivalent iron compound, such as ferric chloride, ferric sulfate, or ferric nitrate, is subjected to a hydrothermal reaction, goethite particles having a plate-like shape are obtained. Examples of alkalis herein include sodium hydroxide, potassium hydroxide, and ammonia, and it is preferable to use sodium hydroxide. Examples of amine compounds (meaning compounds having an amino group in the molecule) include C₂₋₁₀ alcohol amines, such as ethanolamine and N-methyl ethanolamine. In order for a trivalent iron compound to be entirely converted into iron hydroxide, at least three times as much alkali is required relative to iron (by mole). Therefore, the mixing ratio between a trivalent iron compound and an alkali is preferably 1:3 to 1:15 (by mole), for example. In addition, the mixing ratio between an alkali and an amine compound is preferably 1:1 to 1:5 (by weight), for example. The mixing of an alkali and an amine compound with an aqueous solution of a trivalent iron compound may be such that, for example, an aqueous solution of a trivalent iron compound is mixed with an aqueous solution having dissolved therein an alkali and an amine compound with stirring. The temperatures of the two aqueous solutions upon mixing affect the size of goethite particles, eventually the size of ferromagnetic iron oxide particles. For example, in the case where the temperatures of the aqueous solutions are -10 to 30°C, goethite particles, eventually ferromagnetic iron oxide particles, having a plate-like shape with a length of 20 to 200 nm and a length-to-thickness ratio of 1.5 to 30 can be obtained. The length-to-width ratio is 1 to 10, for example. The lower the temperatures, the smaller the length of the resulting particles.

The hydrothermal reaction of a precipitate obtained by mixing an alkali and an amine compound with an aqueous solution of a trivalent iron compound may be such that, for example, the precipitate is placed in a pressure-resistant vessel, such as an autoclave, and subjected to the reaction at 110 to 220°C for 1 to 5 hours. When the temperature of the hydrothermal reaction is too low, particles are less likely to grow into a plate-like shape. Meanwhile, when the temperature is too high, they grow too much, making it difficult to obtain particles of a predetermined size. Incidentally, with respect to the precipitate obtained by mixing an alkali and an amine compound with an aqueous solution of a trivalent iron compound, after the precipitate is formed, the precipitate-containing suspension may be left to age in a room temperature environment (e.g., 10 to 30°C) for about half a day to about two days and then subjected to a hydrothermal reaction; this makes it easy to obtain goethite particles having a plate-like shape of a predetermined size.

The conversion of goethite into an iron oxide may be performed in accordance with a known method. For example, when goethite is heat-oxidized in air at 400 to 700°C for 30 minutes to 5 hours and then heat-reduced in hydrogen gas at 300 to 450°C for 10 minutes to 3 hours, goethite can be converted into magnetite via hematite (α-Fe₂O₃). In addition, when magnetite is heat-oxidized in air at 200 to 300°C for 1 to 30 minutes, magnetite can be converted into gamma iron oxide. When the temperature of heat oxidation is reduced to less than 200°C, for example, an intermediate iron oxide between magnetite and gamma iron oxide can be obtained. Incidentally, in order to prevent the plate-like shape of particles from being changed due to the heating treatment to convert goethite into an iron oxide, it is preferable that the particle surface is previously coated with a SiO₂ film, etc.

Ferromagnetic iron oxide particles thus produced have a plate-like shape with a length of 20 to 200 nm and a length-to-thickness ratio of 1.5 to 30 and have magnetic properties such that the coercivity is 30 to 300 Oe, the saturation magnetization is 20 to 80 emu/g, and the squareness ratio of the magnetic hysteresis loop is 0.20 to 0.50. The coercivity of the ferromagnetic iron oxide particles having a plate-like shape is based on shape magnetic anisotropy. Accordingly, the squareness ratio of the magnetic hysteresis loop is greater as compared with heretofore proposed ferromagnetic iron oxide particles having a spherical or elliptical shape or ferromagnetic iron oxide particles containing foreign elements such as cobalt and thus having increased coercivity based on crystal magnetic anisotropy. Accordingly, the magnetic hysteresis loss is large, indicating excellent heat generation efficiency. In addition, because the particles have a plate-like shape, when they are introduced into the body and accumulated in a cancerous tumor, the area of contact with the cancerous tumor is greater as compared with particles having a spherical or elliptical shape, resulting in excellent ablation efficiency. Accordingly, together with the excellent heat generation efficiency, even higher therapeutic effects can be expected in cancer ablation.

Incidentally, in order to complement or reinforce the magnetic properties of the ferromagnetic iron oxide particles, foreign elements (cobalt, platinum, magnesium, zinc, nickel, titanium, etc.) may be added to the ferromagnetic iron oxide particles. For example, when magnesium is added in an amount of 0.5 to 5 at% of iron, the rising of the magnetic hysteresis loop can be sharpened. As a result, magnetization easily occurs in response to an alternating current magnetic field, and the area of the hysteresis loop increases, whereby the heat generation efficiency can be further enhanced. Incidentally, it is preferable that the amount of foreign elements added is 0.1 to 5.0 at% of iron. When the amount is too small, the desired effects are less likely to be obtained, while when it is too large, in the production of goethite particles, the particles are less likely to grow into a plate-like shape.

It is preferable that the ferromagnetic iron oxide particles have a BET specific surface area of 30 m²/g or more. When the BET specific surface area is 30 m²/g or more, the particles have on the surface thereof enough binding points to a block copolymer of polyethylene glycol and polystyrene having a phosphorous acid group in the side chain, whereby the block copolymer can be bound firmly and abundantly. The reason why the BET specific surface area of the ferromagnetic iron oxide particles can be more than 30 m²/g appears to be that, in addition to the plate-like shape of the particles, the particle surface has a large number of pores (generally 10 nm or less in size) and/or asperities resulting from the dehydration reaction of goethite particles. The upper limit of the BET specific surface area is not particularly limited, but is preferably 150 m²/g. When the BET specific surface area is more than 150 m²/g, the magnetic properties may be deteriorated, resulting in the deterioration of heat generation properties.

Next, a block copolymer of polyethylene glycol and polystyrene having a phosphorous acid group in the side chain, which is to be bound to the surface of the ferromagnetic iron oxide particles having a plate-like shape, will be described.

The block copolymer can be structurally roughly divided into a polyethylene glycol moiety and a polystyrene moiety having a phosphorous acid group in the side chain. Because of the presence of at least one phosphorous acid group in the polystyrene moiety, the block copolymer can be bound to the surface of ferromagnetic iron oxide particles through a firm Fe-O-P bond. Then, the polyethylene glycol moiety of the block copolymer thus bound to the particle surface creates, on the particle surface, a steric hindrance enough to sufficiently block the magnetic cohesive force exerted between particles. In addition, because the block copolymer does not have electrical charges in the molecule, the adhesion of ions to the particle surface is inhibited, and thus the cohesive force due to electrical charges can also be blocked. As a result, excellent compatibility with a medium for injection can be provided. The block copolymer also prevents in vivo the direct contact of ferromagnetic iron oxide particles with various biological components or the adsorption of biological components on the particle surface. In addition, because of its excellent biocompatibility, the block copolymer contributes to the avoidance of the deterioration of particle accumulation properties in cancer cells due to the particles being captured as foreign substances by the liver. Further, the binding of the block copolymer to the surface does not impair the magnetic properties or heat generation properties of the ferromagnetic iron oxide particles.

A specific example of the block copolymer of polyethylene glycol and polystyrene having a phosphorous acid group in the side chain is a polyethylene glycol-b-poly(4-vinylbenzylphosphonate) (hereinafter abbreviated to "PEG-b-PVBP") represented by the following general formula (I).

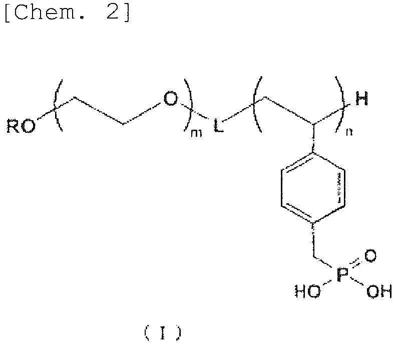

In the formula, R is an optionally substituted alkyl group, L is a linking group selected from the group consisting of -(CH₂)ₗS-, -CO(CH₂)ₗS-, -COO(CH₂)ₗS-, and -COC(CH₃)₂-, 1 is an integer of 1 to 5, m is an integer of 5 to 500, and n is an integer of 2 to 30.

Alkyl groups in optionally substituted alkyl groups herein are C₁₋₁₂ linear or branched alkyl groups, for example. Specific examples thereof include a methyl group, an ethyl group, a propyl group, an isopropyl group, a butyl group, an isobutyl group, a sec-butyl group, a tert-butyl group, a pentyl group, an isopentyl group, a neopentyl group, a hexyl group, an isohexyl group, a heptyl group, and a dodecyl group. Examples of substituents optionally present on the alkyl group include a lower alkoxy group and an R¹R²CH- group. Lower alkoxy groups are C₁₋₄ linear or branched alkoxy groups, for example. Specific examples thereof include a methoxy group, an ethoxy group, a propoxy group, an isopropoxy group, a butoxy group, an isobutoxy group, a sec-butoxy group, and a tert-butoxy group. In an R¹R²CH- group, R¹ and R² are each independently a lower alkoxy group (as defined above), or R¹ and R² together form -O(CH₂)₂O-, -O(CH₂)₃O-, or -O(CH₂)₄O-.

PEG-b-PVBP represented by the above general formula (I) is known as a surface modifier for superparamagnetic iron oxide particles, for example. K. Uj iie et al. , Colloids and Surfaces B: Biointerfaces, 88, 771-778 (2011), states that superparamagnetic iron oxide particles surface-modified with PEG-b-PVBP can be used as an MRI sensitizer. However, the surface modification of ferromagnetic iron oxide particles with PEG-b-PVBP has not been reported.

PEG-b-PVBP can be prepared in accordance with the method of Ujiie et al., in which polyethylene glycol is block-polymerized with polychloromethylstyrene to give polyethylene glycol-b-polychloromethylstyrene, and then chloro groups are converted into phosphonate groups. It is preferable that PEG-b-PVBP has a number average molecular weight of 1,000 to 30, 000. When the number average molecular weight is less than 1,000, the number of phosphonate groups in the molecule is small, and this may cause a problem in that the block copolymer cannot be firmly and abundantly bound to the surface of ferromagnetic iron oxide particles. Meanwhile, when it is more than 30,000, this may cause a problem in that the steric hindrance created by one molecule on the particle surface is too large, resulting in a reduced number of bound molecules.

As a method for binding PEG-b-PVBP to the surface of ferromagnetic iron oxide particles having a plate-like shape, a method in which PEG-b-PVBP is dissolved in a dispersion of the particles in water and/or a hydrophilic solvent as a dispersion medium can be mentioned. In Ujiie et al., which describes superparamagnetic iron oxide particles having PEG-b-PVBP bound to the surface thereof, PEG-b-PVBP is dissolved in an aqueous solution of ferrous chloride and ferric chloride, followed by the addition of ammonia, whereby particles are produced, and also PEG-b-PVBP is bound to the surface thereof at the same time. However, this method cannot be employed as a method for binding PEG-b-PVBP to the surface of ferromagnetic iron oxide particles having a plate-like shape. This is because, as mentioned above, a hydrothermal reaction or heating treatment at 100°C or more is necessary to obtain such particles, and PEG-b-PVBP is exposed to high temperatures at that stage and thus altered or decomposed.

Examples of hydrophilic solvents that can be used for dispersing ferromagnetic iron oxide particles having a plate-like shape include alcohols such as methanol and ethanol, acetone, and acetonitrile. It is preferable that the amount of PEG-b-PVBP dissolved in a dispersion of particles in water and/or a hydrophilic solvent is about 0.1 to about 10 times the amount of particles (by weight). When the amount of PEG-b-PVBP dissolved is too small, PEG-b-PVBP may not be abundantly bound to the particle surface. Meanwhile, when the amount is too large, the amount of PEG-b-PVBP in the dispersion is too large, and the binding between the particles and PEG-b-PVBP may be rather discouraged. It is preferable that the binding of PEG-b-PVBP to the particle surface is performed at a temperature of 30 to 60°C for 1 to 5 days. When the temperature is too low, the binding between the particles and PEG-b-PVBP may not proceed smoothly. Meanwhile, when the temperature is too high, PEG-b-PVBP may be altered or decomposed. Incidentally, in order for the binding between ferromagnetic iron oxide particles having a plate-like shape and PEG-b-PVBP to proceed smoothly, a silane coupling agent, which is a surface modifier, may be previously bound to the particle surface, or various dispersants may be added to the dispersion of particles in water and/or a hydrophilic solvent. Examples of dispersants include polycarboxylic acid dispersants, polyethylene glycol dispersants, naphthalenesulfonic acid formalin condensate dispersants, alkyl sulfonate dispersants, quaternary ammonium dispersants, higher alcohol alkylene oxide dispersants, and polyphosphoric acid dispersants (it is also possible to use a silane coupling agent as a dispersant).

After binding PEG-b-PVBP to the surface of ferromagnetic iron oxide particles having a plate-like shape, precipitates, if any, are removed, and further the dispersion medium is removed. As a result, the powdery surface-modified iron oxide particles for cancer ablation of the present invention, which have excellent dispersion stability in a medium for injection while maintaining the excellent heat generation properties of the ferromagnetic iron oxide particles having a plate-like shape, can be obtained. The surface-modified iron oxide particles for cancer ablation of the present invention thus obtained are, for the purpose of accumulation in target cancer cells, dispersed in a media for injection such as water, physiological saline, or phosphate buffered saline at a concentration of 0.01 to 10 wt%, for example, and thus provided as the surface-modified iron oxide particle dispersion composition for cancer ablation of the present invention. The composition is further diluted with a medium for injection as necessary, and intravenously administered to humans or other mammals. However, the dosage form of the surface-modified iron oxide particle dispersion composition for cancer ablation of the present invention is not limited to intravenous administration, and it may also be directly injected into the lesion (local administration). An alternating current magnetic field is externally applied to the ferromagnetic iron oxide particles thus accumulated in cancer cells. The application may be performed, for example, for 1 to 60 minutes each time at a frequency of 10 to 200 kHz and a maximum applied magnetic field of 100 to 1,000 Oe. Incidentally, at the time of dispersing the surface-modified iron oxide particles for cancer ablation of the present invention in a medium for injection, known additives such as isotonizing agents and stabilizers may be added as necessary. In addition, at the time of dispersing the surface-modified iron oxide particles for cancer ablation of the present invention in a medium for injection, a polyalcohol such as polyethylene glycol may be added in an amount of 10 to 1,000% by weight of the particles, for example, to further enhance the dispersibility of the particles in the medium for injection.

### Examples

Hereinafter, the present invention will be described in detail with reference to examples. However, the present invention should not be construed as being limited to the following description.

### Example 1: Surface-Modified Iron Oxide Particles for Cancer Ablation of the Present Invention (No. 1)

### <A> Production of Ferromagnetic Iron Oxide Particles having Plate-Like Shape

### (Step A-1)

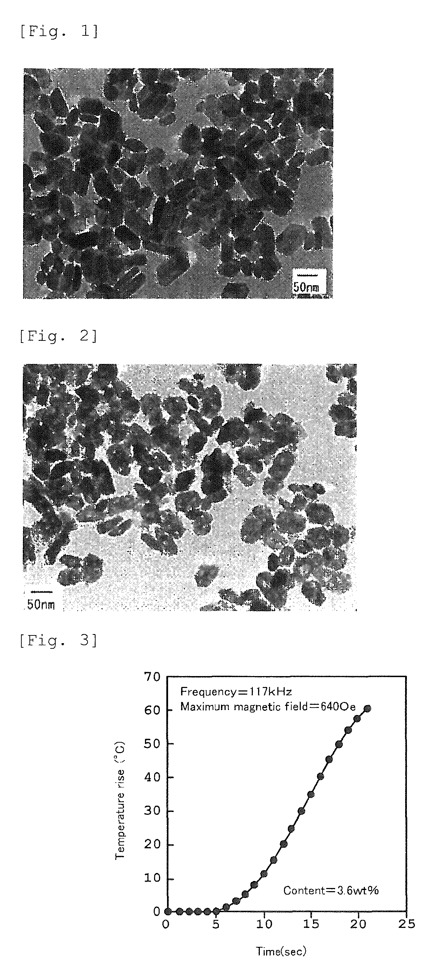

30 g of ferric chloride was dissolved in 450 g of water. Meanwhile, 45 g of sodium hydroxide and 90 g of ethanolamine were dissolved in 900 g of water. The aqueous solution of ferric chloride was cooled to -2.1°C in a freezer, and the aqueous solution of sodium hydroxide and ethanolamine was cooled to -5.4°C in a freezer. Each aqueous solution has dissolved therein a large amount of ions, and thus the freezing point is low. Therefore, they were cooled to a temperature below 0°C at which freezing did not occur. Next, while stirring the latter aqueous solution in a room temperature environment, the former aqueous solution was added dropwise thereto. Stirring was continued for 30 minutes to give an iron hydroxide-containing precipitate. The temperature of the suspension immediately after dropwise mixing was -2.8°C, and the temperature of the suspension after mixing for 30 minutes was 1.8°C. As mentioned above, the size of finally obtained ferromagnetic iron oxide particles having a plate-like shape is almost determined by the temperatures of the two aqueous solutions upon mixing. The lower the temperatures, the smaller the particle size. Next, the precipitate was allowed to age in a room temperature environment for one day. Subsequently, the precipitate was isolated, placed in an autoclave, and subjected to a hydrothermal reaction at 130°C for 2 hours to give goethite particles (goethite was identified by X-ray diffraction structural analysis). Fig. 1 shows a transmission electron microscope (TEM) photograph of the obtained goethite particles. The goethite particles obtained by this method had an average length of about 50 nm and an average width of about 25 nm, and the length-to-width ratio was about 2. In addition, as a result of observation with a scanning electron microscope (SEM), it was confirmed that the particles had a plate-like shape about 5 nm in thickness (the length-to-thickness ratio was about 10).

### (Step A-2)

The goethite particles obtained in Step A-1 were washed with pure water to remove sodium ions and ethanolamine, and then suspended in pure water without drying. Sodium silicate was dissolved in the suspension in a SiO₂ amount of 10% by weight of the goethite particles. Because of the strong alkalinity of sodium silicate, the suspension after dissolving sodium silicate was about pH 10. While stirring the suspension having dissolved therein sodium silicate, dilute hydrochloric acid was added for neutralization to pH 7 to 8. Stirring was further continued for 3 hours to coat the surface of the goethite particles with a SiO₂ film.

### (Step A-3)

The goethite particles surface-coated with a SiO₂ film obtained in Step A-2 were washed with pure water, dried, and then heat-oxidized in air at 500°C for 1 hour to convert the goethite particles into hematite particles. Next, the hematite particles were heat-reduced in hydrogen gas at 380°C for 1 hour to convert the hematite particles into magnetite particles (magnetite was identified by X-ray diffraction structural analysis).

### (Step A-4)

The magnetite particles obtained in Step A-3 were heat-oxidized in air at 250°C for 10 minutes to convert the magnetite particles into gamma iron oxide particles (gamma iron oxide was identified by X-ray diffraction structural analysis). Fig. 2 shows a TEM photograph of the obtained gamma iron oxide particles. The gamma iron oxide particles obtained by this method had an average length of about 43 nm and an average width of about 20 nm, and the length-to-width ratio was about 2. In addition, as a result of observation with a SEM, it was confirmed that the particles had a plate-like shape about 5 nm in thickness (the length-to-thickness ratio was about 8). The BET specific surface area of the gamma iron oxide particles measured by a nitrogen adsorption method was 65 m²/g. The reason why the gamma iron oxide particles had such a large BET specific surface area appeared to be that, in addition to the plate-like shape of the particles, as is clear from Fig. 2, the particle surface had a large number of pores (generally 10 nm or less in size) and asperities resulting from the dehydration reaction of goethite particles.

### <B> Magnetic Properties and Heat Generation Properties of Ferromagnetic Iron Oxide Particles having Plate-Like Shape

The gamma iron oxide particles having a plate-like shape produced in <A> had a coercivity of 155 Oe and a saturation magnetization of 48.6 emu/g, and the squareness ratio of the magnetic hysteresis loop was 0.38 (measured at a maximum applied magnetic field of 13,000 Oe using a vibrating sample magnetometer manufactured by DMS). In accordance with the method of E. Kita et al. (J. Phys. D. 43 (2010) 474011), the heat generation properties of the gamma iron oxide particles were examined under the following conditions: the concentration of particles dispersed in pure water: 3.6 wt%, frequency: 117 kHz, maximum applied magnetic field: 640 Oe. The results are shown in Fig. 3. As is clear from Fig. 3, the gamma iron oxide particles had excellent heat generation properties, and the maximum loss power (SLP: specific loss power, E. Kita et al., J. Appl. Phys. 107, 09B321 (2010)) was 870 W/g. Incidentally, as a conventional example, magnetite particles containing about 2 at% cobalt and having a spherical shape about 20 nm in diameter (produced in accordance with the method of Patent Document 1) had a coercivity of about 150 Oe and a saturation magnetization of about 78 emu/g, and the squareness ratio of the magnetic hysteresis loop was about 0.23. The maximum loss power under the same conditions as above was 200 to 300 W/g. From the above results, although the gamma iron oxide particles produced in <A> and the cobalt-containing magnetite particles as a conventional example are both ferromagnetic iron oxide particles having a spinel crystal structure, because of the difference in shape, the magnetic properties were different, and this difference led to a difference in heat generation efficiency. Accordingly, the advantage of ferromagnetic iron oxide particles having a plate-like shape was confirmed. In addition, it appeared that the large number of pores and asperities present on the surface of the gamma iron oxide particles produced in <A> would contribute to providing enough binding points to a block copolymer of polyethylene glycol and polystyrene having a phosphorous acid group in the side chain, allowing the block copolymer to be bound firmly and abundantly.

### <C> Production of Block Copolymer of Polyethylene Glycol and Polystyrene having Phosphorous Acid Group in the Side Chain

In accordance with the method of Uj iie et al. , PEG-b-PVBP was produced by the following steps, as a block copolymer of polyethylene glycol and polystyrene having a phosphorous acid group in the side chain.

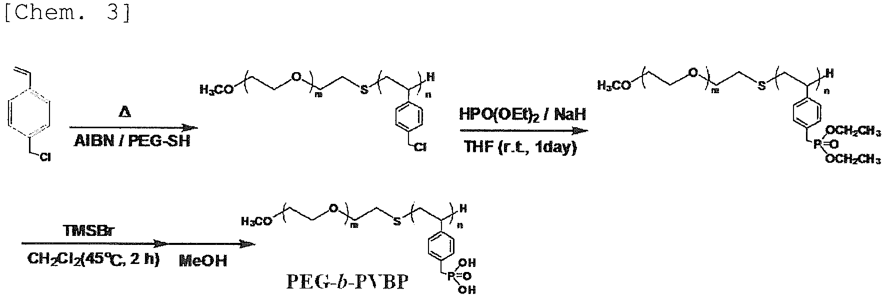

### (Step C-1)

Polyethylene glycol was block-polymerized with polychloromethylstyrene to synthesize polyethylene glycol-b-polychloromethylstyrene (hereinafter abbreviated to "PEG-b-PCMS"). Specifically, in a nitrogen atmosphere, 1 g of MeO-PEG-SH having an average molecular weight of 5, 000 (the number of units (m) : about 110, lyophilized from benzene before use), 16. 5 mg of azobisisobutyronitrile, and 20 mL of toluene were placed in a reaction vessel. Further, 1.39 mL of chloromethylstyrene was added thereto, followed by stirring in an oil bath at 60°C for 24 hours to cause block polymerization. The product was precipitated from diethyl ether, filtered, then dissolved in benzene and lyophilized, and recovered as a powder. The product was identified as PEG-b-PCMS by structural analysis by NMR measurement.

### (Step C-2)

Chloro groups of PEG-b-PCMS were converted into diethyl phosphonate groups to synthesize polyethylene glycol-b-poly(4-vinylbenzyl diethyl phosphonate) (hereinafter abbreviated to "PEG-b-PVBPE"). Specifically, in a nitrogen atmosphere, 400 mg of NaH, 10 mg of NaI, and 10 mL of THF were placed in a reaction vessel, and 1.45 mL of dimethyl phosphite was further added in an ice bath. Meanwhile, in another reaction vessel in an argon atmosphere, 1 g of PEG-b-PCMS synthesized in Step C-1 was dissolved in 5 mL of THF. The latter was slowly added dropwise to the former in an ice bath using a syringe, and the mixture was allowed to react at room temperature overnight. The solvent was evaporated, and then the product was dialyzed against methanol, dissolved in benzene and lyophilized, and recovered as a powder. The product was identified as PEG-b-PVBPE by structural analysis by NMR measurement.

### (Step C-3)

Diethyl phosphonate groups of PEG-b-PVBPE were converted into phosphonate groups to synthesize PEG-b-PVBP, the target product. Specifically, in a reaction vessel in a nitrogen atmosphere, 5 mL of dichloromethane was added to 1.2 g of PEG-b-PVBPE synthesized in Step C-2. Further, 1.46 mL of bromotrimethylsilane was added thereto, followed by refluxing at 45°C for 2 hours in an oil bath. Subsequently, the solvent was evaporated, and methanol was added and stirred at room temperature for 15 hours. The product was dialyzed against methanol, then dissolved in benzene and lyophilized, and recovered as a powder. The product was identified as PEG-b-PVBP by structural analysis by NMR measurement. The number average molecular weight of PEG-b-PVBP thus obtained was 6,900, and the molecular weight distribution was 1.25 (by gel permeation chromatography). In addition, from the peak of each signal in NMR measurement, the number of 4-vinylbenzylphosphonate units (n) was about 15.

### <D> Production of Surface-Modified Iron Oxide Particles for Cancer Ablation of the Present Invention

0.8 mg of the gamma iron oxide particles having a plate-like shape produced in <A> were placed in a 100-cc screw tube, and 80 g of ethanol was further added, followed by ultrasonic dispersion for about 4 hours. The obtained dispersion was allowed to stand for one week. As a result, some of the particles sedimented at the bottom of the screw tube. The supernatant in the screw tube was collected to give a dispersion of gamma iron oxide particles. The content of gamma iron oxide particles in the dispersion was 82 wt%. Next, 2.0 g of the dispersion was placed in a 10-cc screw tube, and then PEG-b-PVBP produced in <C> was added in an amount of 50% by weight of the gamma iron oxide particles and dissolved with ultrasonic dispersion. The obtained gamma iron oxide particle dispersion having dissolved therein PEG-b-PVBP was placed in a screw tube, and the tube was capped and allowed to stand at 50°C for two days, thereby binding PEG-b-PVBP to the surface of the gamma iron oxide particles. The screw tube was uncapped and placed in a desiccator at 30°C, and, while stirring the dispersion, ethanol was removed by drying to give powdery gamma iron oxide particles having PEG-b-PVBP bound to the surface thereof. The magnetic properties and heat generation properties of the gamma iron oxide particles having PEG-b-PVBP bound to the surface thereof were evaluated in the same manner as in <B>. As a result, the properties were not substantially different from those of the gamma iron oxide particles before binding PEG-b-PVBP to the surface.

### <E> Dispersion Stability of Surface-Modified Iron Oxide Particles for Cancer Ablation of the Present Invention in Physiological Saline

100 mg of the gamma iron oxide particles having PEG-b-PVBP bound to the surface thereof produced in <D> were placed in a 100-cc screw tube, and 30 g of physiological saline was further added, followed by ultrasonic dispersion for about 1 hour. The obtained dispersion was allowed to stand for two days. As a result, some of the particles sedimented at the bottom of the screw tube. The supernatant in the screw tube was collected to give a dispersion of gamma iron oxide particles having PEG-b-PVBP bound to the surface thereof. The content of gamma iron oxide particles having PEG-b-PVBP bound to the surface thereof in the dispersion was 0.2 wt%. The dispersion was diluted with physiological saline to prepare several dispersions with the content of gamma iron oxide particles having PEG-b-PVBP bound to the surface thereof being within a range of 0.001 to 0.2 w%, and the dispersion stability of each dispersion was examined. As a result, in any dispersion, the formation of sediments was not seen even in one month after preparation. Thus, they had extremely excellent dispersion stability.

### Example 2: Surface-Modified Iron Oxide Particles for Cancer Ablation of the Present Invention (No. 2)

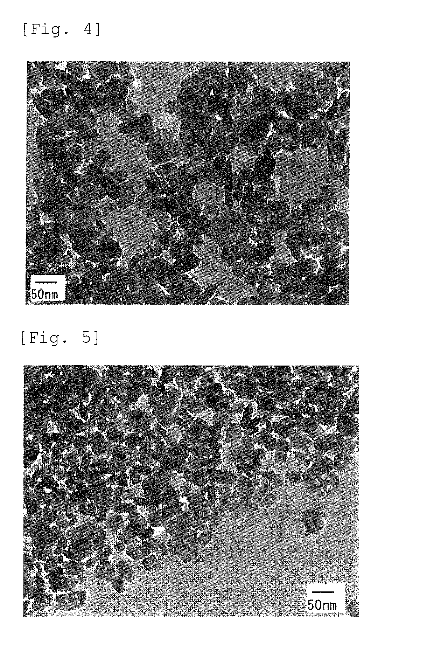

30 g of ferric chloride was dissolved in 450 g of water. Meanwhile, 45 g of sodium hydroxide and 90 g of ethanolamine were dissolved in 900 g of water. The aqueous solution of ferric chloride was cooled to -3.2°C in a freezer, and the aqueous solution of sodium hydroxide and ethanolamine was cooled to -7.2°C in a freezer. Next, while stirring the latter aqueous solution in a room temperature environment, the former aqueous solution was added dropwise thereto. Stirring was continued for 30 minutes to give an iron hydroxide-containing precipitate. The temperature of the suspension immediately after dropwise mixing was -3.6°C, and the temperature of the suspension after mixing for 30 minutes was -0.2°C. Next, the precipitate was allowed to age in a room temperature environment for one day. Subsequently, the precipitate was isolated, placed in an autoclave, and subjected to a hydrothermal reaction at 130°C for 2 hours to give goethite particles (goethite was identified by X-ray diffraction structural analysis). Fig. 4 shows a TEM photograph of the obtained goethite particles. The goethite particles obtained by this method had an average length of about 40 nm and an average width of about 20 nm, and the length-to-width ratio was about 2. In addition, as a result of observation with a scanning electron microscope (SEM), it was confirmed that the particles had a plate-like shape about 5 nm in thickness (the length-to-thickness ratio was about 8).

Subsequently, gamma iron oxide particles were obtained in the same manner as in <A>, Steps A-2 to A-4, of Example 1. Fig. 5 shows a TEM photograph of the obtained gamma iron oxide particles. The gamma iron oxide particles obtained by this method had an average length of about 35 nm and an average width of about 18 nm, and the length-to-width ratio was about 2. In addition, as a result of observation with a SEM, it was confirmed that the particles had a plate-like shape about 5 nm in thickness, and a large number of pores (generally 10 nm or less in size) and asperities resulting from the dehydration reaction of goethite particles were present on the particle surface (the length-to-thickness ratio was about 7). The BET specific surface area of the gamma iron oxide particles measured by a nitrogen adsorption method was 78 m²/g. The gamma iron oxide particles had a coercivity of 95 Oe and a saturation magnetization of 47.2 emu/g, and the squareness ratio of the magnetic hysteresis loop was 0.33 (measured at a maximum applied magnetic field of 13,000 Oe using a vibrating sample magnetometer manufactured by DMS). In accordance with the method of E. Kita et al. (J. Phys. D. 43 (2010) 474011), the heat generation properties of the gamma iron oxide magnetic particles were examined under the following conditions: the concentration of particles dispersed in pure water: 3.6 wt%, frequency: 117 kHz, maximum applied magnetic field: 640 Oe. As a result, the maximum loss power (SLP: specific loss power, E. Kita et al., J. Appl. Phys. 107, 09B321 (2010)) was 580 W/g. Incidentally, the magnetite particles containing about 2 at% cobalt and having a spherical shape about 20 nm in diameter described in <B> of Example 1 had a coercivity of about 150 Oe and a saturation magnetization of about 78 emu/g, and the squareness ratio of the magnetic hysteresis loop was about 0.23. The maximum loss power under the same conditions as above was 200 to 300 W/g. This shows that although the coercivity of the gamma iron oxide particles having a plate-like shape was about 2/3 that of the cobalt-containing magnetite particles having a spherical shape, the maximum loss power was about twice as much.

Next, in the same manner as in <D> of Example 1, powdery gamma iron oxide particles having PEG-b-PVBP bound to the surface thereof were obtained. Similarly to the gamma iron oxide particles having PEG-b-PVBP bound to the surface thereof obtained in Example 1, the gamma iron oxide particles having PEG-b-PVBP bound to the surface thereof had extremely excellent dispersion stability while maintaining the magnetic properties and heat generation properties of the gamma iron oxide particles before binding PEG-b-PVBP to the surface.

### Example 3: Surface-Modified Iron Oxide Particles for Cancer Ablation of the Present Invention (No. 3)

PEG-b-PVBP was bound to the surface of gamma iron oxide particles in the same manner as in <D> of Example 1, except that at the time of binding PEG-b-PVBP to the surface of gamma iron oxide particles in <D> of Example 1, PEG-b-PVBP was added in an amount of 100% by weight of the gamma iron oxide particles, and that a screw tube containing a gamma iron oxide particle dispersion having dissolved therein PEG-b-PVBP was capped and allowed to stand at 40°C for two days. Similarly to the gamma iron oxide particles having PEG-b-PVBP bound to the surface thereof obtained in Example 1, the gamma iron oxide particles having PEG-b-PVBP bound to the surface thereof had extremely excellent dispersion stability while maintaining the magnetic properties and heat generation properties of the gamma iron oxide particles before binding PEG-b-PVBP to the surface.

### Example 4: Surface-Modified Iron Oxide Particles for Cancer Ablation of the Present Invention (No. 4)

PEG-b-PVBP was bound to the surface of gamma iron oxide particles in the same manner as in <D> of Example 1, except that 2.0 g of the dispersion of gamma iron oxide particles in ethanol described in <D> of Example 1 was placed in a 10-cc screw tube, then a silane coupling agent (3-methacryloxy propyl methyl dimethoxysilane manufactured by Shin-Etsu Chemical Co. , Ltd.) was added as a surface modifier in an amount of 20% by weight of the gamma iron oxide particles and dissolved with ultrasonic dispersion, the obtained gamma iron oxide particle dispersion having dissolved therein the silane coupling agent was placed in a screw tube, and the tube was capped and allowed to stand at 50°C for two days, thereby binding the silane coupling agent to the surface of the gamma iron oxide particles, followed by the addition of PEG-b-PVBP. Similarly to the gamma iron oxide particles having PEG-b-PVBP bound to the surface thereof obtained in Example 1, the gamma iron oxide particles having PEG-b-PVBP bound to the surface thereof had extremely excellent dispersion stability while maintaining the magnetic properties and heat generation properties of the gamma iron oxide particles before binding PEG-b-PVBP to the surface.

### Example 5: Surface-Modified Iron Oxide Particles for Cancer Ablation of the Present Invention (No. 5)

PEG-b-PVBP was bound to the surface of gamma iron oxide particles in the same manner as in <D> of Example 1, except that 2.0 g of the dispersion of gamma iron oxide particles in ethanol described in <D> of Example 1 was placed in a 10-cc screw tube, then a silane coupling agent (3-methacryloxy propyl methyl dimethoxysilane manufactured by Shin-Etsu Chemical Co. , Ltd.) was added as a surface modifier in an amount of 20% by weight of the gamma iron oxide particles and dissolved with ultrasonic dispersion, the obtained gamma iron oxide particle dispersion having dissolved therein the silane coupling agent was placed in a screw tube, and the tube was capped and allowed to stand at 50°C for two days, thereby binding the silane coupling agent to the surface of the gamma iron oxide particles, followed by the addition of PEG-b-PVBP in an amount of 100% by weight of the gamma iron oxide particles; and also that a screw tube containing the gamma iron oxide particle dispersion having dissolved therein PEG-b-PVBP was capped and allowed to stand at 40°C for two days. Similarly to the gamma iron oxide particles having PEG-b-PVBP bound to the surface thereof obtained in Example 1, the gamma iron oxide particles having PEG-b-PVBP bound to the surface thereof had extremely excellent dispersion stability while maintaining the magnetic properties and heat generation properties of the gamma iron oxide particles before binding PEG-b-PVBP to the surface.

### Comparative Example 1: Dispersion Stability of Ferromagnetic Iron Oxide Particles having Plate-Like Shape in Physiological Saline

2.0 g of the dispersion of gamma iron oxide particles in ethanol described in <D> of Example 1 was placed in a 10-cc screw tube. Subsequently, without dissolving PEG-b-PVBP, the screw tube was placed in a desiccator at 25°C with the cap removed, and, while stirring the dispersion, ethanol was removed by drying. 60 mg of the powdery gamma iron oxide particles thus obtained were placed in a 100-cc screw tube, and 30 g of physiological saline was further added, followed by ultrasonic dispersion for about 1 hour. In the obtained dispersion, particles started to sediment at the bottom of the screw tube immediately after the completion of ultrasonic dispersion, and all the particles sedimented in a few minutes (the content of gamma iron oxide particles in the supernatant was 0 wt%). This result shows that the gamma iron oxide particles produced in <A> of Example 1 have excellent dispersion stability in ethanol but have no dispersion stability in physiological saline.

### Comparative Example 2: Dispersion Stability of Ferromagnetic Iron Oxide Particles having Plate-Like Shape with Silane Coupling Agent Bound to Surface Thereof in Physiological Saline

2.0 g of the dispersion of gamma iron oxide particles in ethanol described in <D> of Example 1 was placed in a 10-cc screw tube. Subsequently, a silane coupling agent (3-methacryloxy propyl methyl dimethoxysilane manufactured by Shin-Etsu Chemical Co. , Ltd.) was added as a surface modifier in an amount of 50% by weight of the gamma iron oxide particles and dissolved with ultrasonic dispersion. The obtained gamma iron oxide particle dispersion having dissolved therein the silane coupling agent was placed in a screw tube, and the tube was capped and allowed to stand at 50°C for two days, thereby binding the silane coupling agent to the surface of the gamma iron oxide particles. The screw tube was uncapped and placed in a desiccator at 25°C, and, while stirring the dispersion, ethanol was removed by drying to give powdery gamma iron oxide particles having a silane coupling agent bound to the surface thereof. The dispersion stability of the gamma iron oxide particles having a silane coupling agent bound to the surface thereof thus obtained in physiological saline was evaluated in the same manner as in Comparative Example 1. As a result, in the obtained dispersion, the sedimentation of particles at the bottom of the screw tube was not seen for about 10 minutes after the completion of ultrasonic dispersion. However, sedimentation started afterward, and all the particles sedimented in 1 hour (the content of gamma iron oxide particles in the supernatant was 0 wt%). This result shows that even when the gamma iron oxide particles produced in <A> of Example 1 were surface-modified with a silane coupling agent, dispersion stability in physiological saline was hardly improved.

### Comparative Example 3: Dispersion Stability of Ferromagnetic Iron Oxide Particles having Spherical Shape in Physiological Saline

The dispersion stability of the magnetite particles containing about 2 at% cobalt and having a spherical shape about 20 nm in diameter described in <B> of Example 1 in physiological saline was evaluated in the same manner as in Comparative Example 1. The result was the same as in the case of gamma iron oxide particles having a plate-like shape, showing that the particles have excellent dispersion stability in ethanol but have no dispersion stability in physiological saline.

### Comparative Example 4: Dispersion Stability of Ferromagnetic Iron Oxide Particles having Spherical Shape with Silane Coupling Agent Bound to Surface Thereof in Physiological Saline

Powdery magnetite particles having a silane coupling agent bound to the surface thereof were obtained in the same manner as in Comparative Example 2, except that the magnetite particles containing about 2 at% cobalt and having a spherical shape about 20 nm in diameter described in <B> of Example 1 were used in place of the gamma iron oxide particles produced in <A> of Example 1. The dispersion stability of the particles in physiological saline was evaluated in the same manner as in Comparative Example 1. As a result, although dispersion stability was improved by binding a silane coupling agent to the surface, all the particles sedimented in 3 hours.

### Comparative Example 5: Dispersion Stability of Ferromagnetic Iron Oxide Particles having Spherical Shape with PEG-b-PVBP Bound to Surface Thereof in Physiological Saline

Powdery magnetite particles having PEG-b-PVBP bound to the surface thereof were obtained in the same manner as in <D> of Example 1, except that the magnetite particles containing about 2 at% cobalt and having a spherical shape about 20 nm in diameter described in <B> of Example 1 were used in place of the gamma iron oxide particles produced in <A> of Example 1. The dispersion stability of the particles in physiological saline was evaluated in the same manner as in Comparative Example 1. As a result, although dispersion stability was further improved by binding PEG-b-PVBP to the surface, all the particles sedimented in 6 hours.

### Summary of Examples and Comparative Examples:

PEG-b-PVBP as a surface modifier was not able to impart excellent dispersion stability in physiological saline to ferromagnetic iron oxide particles of any shape. Although excellent dispersion stability was imparted to ferromagnetic iron oxide particles having a plate-like shape, the degree of stability imparted to ferromagnetic iron oxide particles having a spherical shape was not enough for practical applications. This shows that the effects of the combination of ferromagnetic iron oxide particles having a plate-like shape and PEG-b-PVBP are specific to this combination.

### Preparation Example 1:

The gamma iron oxide particles produced in Example 1 were dispersed in pure water at a concentration of 0.05 to 5 wt% to prepare a dispersion composition for intravenous administration.

### Industrial Applicability

According to the present invention, surface-modified ferromagnetic iron oxide particles suitable for electromagnetic-induction cancer ablation, which have excellent heat generation properties and also have excellent dispersion stability in a medium for injection, can be provided. In this respect, the present invention is industrially applicable.

## Claims

1. Surface-modified iron oxide particles for cancer ablation, **characterized in that** a block copolymer of polyethylene glycol and polystyrene having a phosphorous acid group in the side chain is bound to the surface of ferromagnetic iron oxide particles having a plate-like shape with a length of 20 to 200 nm and a length-to-thickness ratio of 1.5 to 30 and having magnetic properties such that the coercivity is 30 to 300 Oe, the saturation magnetization is 20 to 80 emu/g, and the squareness ratio of the magnetic hysteresis loop is 0.20 to 0.50.

2. The surface-modified iron oxide particles for cancer ablation according to claim 1, **characterized in that** the iron oxide is magnetite, gamma iron oxide, or an intermediate iron oxide between magnetite and gamma iron oxide.

3. The surface-modified iron oxide particles for cancer ablation according to claim 1, **characterized in that** the ferromagnetic iron oxide particles have a BET specific surface area of 30 m²/g or more.

4. The surface-modified iron oxide particles for cancer ablation according to claim 1, **characterized in that** the block copolymer of polyethylene glycol and polystyrene having a phosphorous acid group in the side chain is a polyethylene glycol-b-poly(4-vinylbenzylphosphonate) represented by the following general formula (I): 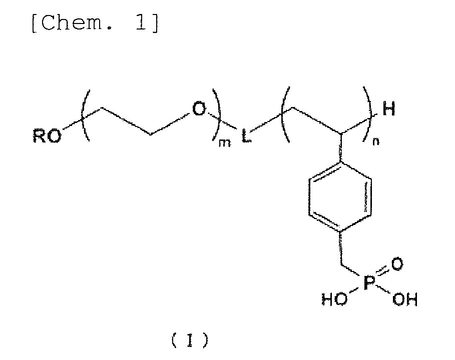 wherein R is an optionally substituted alkyl group, L is a linking group selected from the group consisting of - (CH₂)ₗS-, -CO(CH₂)ₗS-, -COO(CH₂)ₗS-, and -COC(CH₃)₂-, 1 is an integer of 1 to 5, m is an integer of 5 to 500, and n is an integer of 2 to 30.

5. The surface-modified iron oxide particles for cancer ablation according to claim 1, **characterized in that** the block copolymer has a number average molecular weight of 1,000 to 30,000.

6. A method for producing surface-modified iron oxide particles for cancer ablation, **characterized in that** a block copolymer of polyethylene glycol and polystyrene having a phosphorous acid group in the side chain is dissolved in a dispersion of ferromagnetic iron oxide particles in water and/or a hydrophilic solvent as a dispersion medium, thereby binding the block copolymer to the surface of the ferromagnetic iron oxide particles,
the ferromagnetic iron oxide particles having a plate-like shape with a length of 20 to 200 nm and a length-to-thickness ratio of 1.5 to 30 and having magnetic properties such that the coercivity is 30 to 300 Oe, the saturation magnetization is 20 to 80 emu/g, and the squareness ratio of the magnetic hysteresis loop is 0.20 to 0.50.

7. The production method according to claim 6, **characterized in that** the binging of the block copolymer to the surface of the ferromagnetic iron oxide particles is performed at a temperature of 30 to 60°C.

8. The production method according to claim 6, **characterized in that** the ferromagnetic iron oxide particles are produced by subjecting a precipitate obtained by mixing an alkali and an amine compound with an aqueous solution of a trivalent iron compound to a hydrothermal reaction to produce goethite particles, followed by converting goethite into an iron oxide.

9. The production method according to claim 8, **characterized in that** the amine compound is an alcohol amine.

10. A surface-modified iron oxide particle dispersion composition for cancer ablation, **characterized in that** surface-modified iron oxide particles for cancer ablation made of ferromagnetic iron oxide particles having bound to the surface thereof a block copolymer of polyethylene glycol and polystyrene having a phosphorous acid group in the side chain are dispersed in a medium for injection,
the ferromagnetic iron oxide particles having a plate-like shape with a length of 20 to 200 nm and a length-to-thickness ratio of 1.5 to 30 and having magnetic properties such that the coercivity is 30 to 300 Oe, the saturation magnetization is 20 to 80 emu/g, and the squareness ratio of the magnetic hysteresis loop is 0.20 to 0.50.
